# FASCICULE DE BREVET EUROPEEN

(11) **EP 1 406 637 B1**
(45) Date de publication et mention de la délivrance du brevet: **06.06.2007**
(21) Numéro de dépôt: 00949546.6
(22) Date de dépôt: 15.06.2000
(51) Int. Cl.: A61K 31/721, C08B 37/02, A61P 1/04, A61P 17/02, A61P 21/00, A61P 27/00

(54) **COMPOSITIONS PHARMACEUTIQUES A ACTION CICATRISANTE OU ANTI-COMPLEMENTAIRE COMPRENANT UN DERIVE DE DEXTRANE**
PHARMAZEUTISCHE ZUSAMMENSETZUNGEN MIT WUNDHEILENDER UND ANTI-KOMPLEMENTÄRER WIRKUNG DIE EIN DEXTRANDERIVAT ENTHALTEN
PHARMACEUTICAL COMPOSITIONS WITH WOUND HEALING OR ANTI-COMPLEMENTARY ACTIVITY COMPRISING A DEXTRAN DERIVATIVE

(30) Priorité: 16.06.1999 FR 9907636
(43) Date de publication de la demande: 14.04.2004
(73) Titulaire: BIODEX, 69003 Lyon (FR)
(72) Inventeur: DAHRI-CORREIA, Latifa, F-59230 Saint Amand les Eaux (FR); JOZEFONVICZ, Jacqueline, F-60260 Lamorlaye (FR); JOZEFOWICZ, Marcel, F-60260 Lamorlaye (FR); CORREIA, José, F-59230 Saint Amand les Eaux (FR); HUYNH, Rémi, F-59230 Saint Amand les Eaux (FR)
(74) Mandataire: Tripoz, Inès
(86) Numéro de dépôt international: PCT/FR2000/001658
(87) Numéro de publication internationale: WO 2000/076452

(56) Documents cités:
- WO-A-95/26736
- FR-A- 2 555 589
- FR-A- 2 644 066
- FR-A- 2 651 436
- FR-A- 2 657 782
- FR-A- 2 772 382
- MAIGA-REVEL O ET AL: "New investigations on heparin-like derivatized dextrans: CMDBS, synergistic role of benzylamide and sulfate substituents in anticoagulant activity" CARBOHYDRATE POLYMERS,GB,APPLIED SCIENCE PUBLISHERSLTD. BARKING, vol. 32, no. 2, 1 février 1997 (1997-02-01), pages 89-93, XP004063799 ISSN: 0144-8617
- LETOURNEUR, DIDIER ET AL: "Antiproliferative capacity of synthetic dextrans on smooth muscle cell growth: The model of derivatized dextrans as heparin-like polymers" J. BIOMATER. SCI., POLYM. ED. (1993), 4(5), 431-44 , 1993, XP000978203
- DATABASE WPI Section Ch, Week 197505 Derwent Publications Ltd., London, GB; Class B04, AN 1975-08052W XP002157354 & JP 49 025120 A (SEIKAGAKU KOGYO CO LTD), 6 mars 1974 (1974-03-06)
- BLANQUAERT ET AL: "Les CMDBS analogues fonctionnels des heparanes sulfates, utilisés comme agents de la cicatrisation osseuse" ANNALES D'ENDOCRINOLOGIE,FR,MASSON, PARIS, vol. 55, no. 2, 1994, pages 121-123-123, XP002100532 ISSN: 0003-4266
- STOCKHOLM, DANIEL ET AL: "Studies on Calpain Expression during Differentiation of Rat Satellite Cells in Primary Cultures in the Presence of Heparin or a Mimic Compound" EXP. CELL RES. (1999), 252(2), 392-400 , 1999, XP000980365
- MEDDAHI ET AL: "New approaches to tissue regeneration and repair" PATHOLOGY RESEARCH AND PRACTICE,DE,GUSTAV FISCHER, STUTTGART, vol. 190, no. 9/10, 1994, pages 923-928, XP002098415 ISSN: 0344-0338
- GAUTRON ET AL: "Injection of a dextran derivatives in adult rat skeletal muscle accelerates its regeneration" COMPTES RENDUS DES SEANCES DE L'ACADEMIE DES SCIENCES. SERIE III: SCIENCES DE LA VIE,NL,ELSEVIER, AMSTERDAM, vol. 318, no. 6, 1995, pages 671-676, XP002098424 ISSN: 0764-4469
- MAUZAC, M. ET AL: "Anticoagulant activity of dextran derivatives. Part I: synthesis and characterization" BIOMATERIALS (GUILDFORD, ENGL.) (1984), 5(5), 301-4, XP000876841
- CHAUBET F ET AL: "Synthesis and structure-anticoagulant property relationships of functionalized dextrans: CMDBS" CARBOHYDRATE POLYMERS,GB,APPLIED SCIENCE PUBLISHERSLTD. BARKING, vol. 28, no. 2, 1995, pages 145-152, XP004034410 ISSN: 0144-8617

## Description

La présente invention est relative à des compositions pharmaceutiques à action cicatrisante ou anti-complémentaire comprenant au moins un dérivé de dextrane.

Différents dextranes substitués par des chaînes latérales portant des groupes carboxylates et sulfonates ont été décrits. En particulier, des dérivés du dextrane comportant respectivement 83% ou 110 % d'unités substituées par des groupes carboxymétyles, 23 % ou 2,6 % d'unités substituées par des groupes carboxyméthylbenzylamides et 13 % ou 36,5 % d'unités substituées par des groupes sulfonates (groupements sulfonates portés par les unités carboxyméthylbenzylamides), à savoir respectivement le RGTA9 et le RGTA11, ont été décrits pour leur action *in vivo,* chez le rat, sur la réparation cutanée (A. Meddahi *et al.,* Path. Res. Pract., 1994, 190, 923-928 ; A. Meddahi *et al.,* Diabetes & Metabolism (Paris), 1996, 22, 274-278) et sur la régénération musculaire (A. Aamiri *et al.,* Neuroscience Letters, 1995, 201, 243-246 ; J. Gautron *et al.,* C. R. Acad. Sci. Paris, Life sciences, Cell biology, 1995, 318, 671-6 ; A . Aamiri *et al.,* C. R. Acad. Sci. Paris, Life sciences, Neurosciences, 1995, 318, 1037-43).

En ce qui concerne la réparation cutanée, A. Meddahi *et al.* (*ibid*) proposent de combler des plaies cutanées par des pièces de collagène imbibées d'une solution de RGTA9 ou de RGTA11 ; dans ces conditions, une amélioration de la vitesse et de la qualité de la régénération cutanée est constatée. Elle pourrait être expliquée en ce que le RGTA9 ou le RGTA11 piègent, protègent et libèrent les facteurs de croissance endogènes naturellement sécrétés au cours de la cicatrisation cutanée. La protection des facteurs de croissance permettrait d'éviter leur dégradation par les protéases naturelles, préservant ainsi leur aptitude à stimuler la réparation tissulaire.

Dans le domaine de la régénération musculaire, A. Aamiri *et al.* et J. Gautron *et al.* (*ibid*) proposent d'injecter à des rats, dont les muscles rapides (EDL : *Extensor Digitorium Longus*) et/ou lents (*soleus*) ont été écrasés, une solution de RGTA11. Ils constatent une meilleure régénération des muscles suite à cette injection : les muscles traités présentent un plus grand nombre de fibres musculaires et une réinnervation plus rapide.

Toutefois, les RGTA9 et 11 précités, de par leur procédé de préparation, ont l'inconvénient de présenter une distribution irrégulière des groupements chimiques (carboxyméthyles, carboxyméthylbenzylamides et sulfonates) et des chaînes polysaccharidiques, conduisant à un produit final hétérogène, dont il est difficile de contrôler les propriétés.

La demande de brevet français FR 2 651 436 décrit des compositions pharmaceutiques contenant à titre de principe actif des dérivés substitués solubles de dextrane à activité inhibitrice de la prolifération des cellules musculaires lisses, utiles pour la prévention et le traitement des maladies impliquant la prolifération des cellules musculaires lisses. Ces dérivés contiennent 50 à 70 % de motifs méthylcarboxylates et 20 à 40 % de motifs carboxyméthylbenzylamides. Les doses pharmaceutiques utilisées sont comprises entre 50 mg et 5 g pour une administration par voie orale.

Les Inventeurs se sont donc donnés pour but de sélectionner des dérivés de dextrane différents des composés RGTA9 et 11 déjà décrits afin de pourvoir à des compositions pharmaceutiques à action anti-complémentaire ou à action cicatrisante, notamment dans les domaines des cicatrisations cutanée, musculaire, oculaire ou de la muqueuse gastrique, qui répondent mieux au besoin de la pratique, notamment en ce qu'elles présentent une activité accrue, et qui soient aptes à être administrées chez l'homme, et ce sous des formes et à des doses adaptées à une efficacité optimale.

Ainsi, les Inventeurs ont mis au point des compositions pharmaceutiques à action cicatrisante et anti-complémentaire à base de dérivés de dextrane particuliers.

La présente invention a pour objet une composition pharmaceutique à action cicatrisante comprenant :
**(1)** au moins un dérivé de dextrane de formule générale DMCₐB_{b}Su_{c}, dans laquelle :
   D représente une chaîne polysaccharidique, de préférence constituée par des enchaînements d'unités glucosidiques,
   MC représente des groupes méthylcarboxylates,
   B représente des groupes carboxyméthylbenzylamides,
   Su représente des groupes sulfates (sulfatation des fonctions hydroxyles libres portées par les unités glucosidiques),
   a, b et c représentent le degré de substitution (ds), exprimé par rapport au nombre de fonctions hydroxyles libres dans une unité glucosidique du dextrane, respectivement en groupements MC, B et Su ; a étant ≥ à 0,6, b étant ≥ à 0,1 et c étant compris entre 0,1 et 0,5,
**(2)** ainsi qu'au moins un excipient acceptable du point de vue pharmaceutique,
ledit dérivé de dextrane étant présent à une dose unitaire comprise entre 0,1 et 50 mg.

On entend par « excipient » tout adjuvant ou véhicule sans action pharmacologique mais permettant la fabrication, la conservation ou l'administration de la composition pharmaceutique. Tout excipient acceptable du point de vue pharmaceutique, choisi par exemple parmi les excipients couramment utilisés en galénique, peut être utilisé dans la composition pharmaceutique à action cicatrisante selon l'invention.

La composition pharmaceutique selon l'invention comprend ainsi des dérivés de dextrane qui sont significativement différents de ceux décrits dans l'Art antérieur sous les dénominations RGTA9 et 11, et ce notamment en ce qu'ils ne comprennent pas d'unités sulfonates.

Les dérivés du dextrane définis ci-dessus sont considérés comme étant des copolymères constitués par des sous-unités fictives R-OH et R-OX, X pouvant être un groupement méthylcarboxylate (MC), benzylamide (B) ou sulfate (Su), la chaîne polysaccharidique du dextrane non substitué étant considérée comme étant constituée par 300 sous-unités fictives R-OH, au lieu de 100 unités glucosidiques, eu égard au fait qu'une unité glucosidique non substituée comporte trois groupes hydroxyles libres. Ainsi, un dextrane méthylcarboxylate (DMC) avec un degré de substitution (ds) de 1,2 en groupements méthylcarboxylates contient 1,20 groupement substitué (R-MC) et 1,80 groupement hydroxyle libre (R-OH), par unité glucosidique. Il est bien entendu que la somme des degrés de substitution a, b et c, dans la formule générale DMCₐB_{b}Su_{c} définie ci-dessus, est inférieure ou égale à 3.

Les dérivés du dextrane de formule générale DMCₐB_{b}Su_{c}, telle que définie ci-dessus, peuvent être obtenus par un procédé qui comprend les étapes suivantes, comme décrit dans le Brevet français 2 772 382 :
a) **carboxyméthylation** comprenant (i) l'activation d'un dextrane non substitué, par mise en contact dudit dextrane avec un milieu hydro-alcoolique liquide biphasique basique pendant au moins 1 h sous agitation, (ii) addition de l'acide monochloroacétique au produit activé obtenu, à une température comprise entre 40 et 90°C, de préférence à 60°C, le rapport R_{MC}, égal au nombre de moles d'acide monochloracétique/nombre de moles d'OH étant compris entre 0,3 et 2, (iii) isolement et éventuellement purification du dextrane méthylcarboxylate (DMC) obtenu ;
b) **couplage de la benzylamine sur les groupes méthylcarboxylates** (benzylamidification) comprenant (i) la mise en contact, pendant au moins 2 h et en milieu aqueux acide, du DMC obtenu en a) avec une amine primaire (benzylamine), en présence d'une carbodiimide hydrosoluble telle que le 1-cyclohexyl-3-(2-morpholinoéthyl)-carbodümide méta-*p*-toluène sulfonate (CMC) ou le chlorohydrate de 1-éthyl-3-(3-diméthylamino-propyl)-carbodiimide (EDC) comme agent de couplage, à une température comprise entre 0°C et 30°C, le rapport molaire carbodiimide hydrosoluble/MC étant compris entre 0,25 et 2 et le rapport molaire benzylamine/MC étant compris entre 0,25 et 2, (ii) l'isolement du dextrane méthylcarboxyle benzylamide (DMCB) obtenu et éventuellement sa purification ; cette étape, réalisée en milieu homogène et en présence d'une carbodiimide hydrosoluble comme réactif de couplage, permet de maîtriser la réaction et donc la préparation du produit final, ce dernier présentant une homogénéité de la distribution des tailles de chaînes, illustrée par un profil d'élution de type gaussien symétrique en chromatographie d'exclusion stérique haute performance, et une homogénéité de la distribution des groupements chimiques chargés, illustrée par un profil d'élution à un seul pic symétrique en chromatographie d'échange d'ions basse pression ; puis
c) **sulfatation** comprenant (i) la formation d'un sel de trialkylammonium du DMCB obtenu en b), (ii) la solubilisation du sel obtenu dans un solvant polaire anhydre, généralement une base de Lewis (donneur d'électron), telle que le diméthylsulfoxyde (DMSO) ou la diméthylformamide (DMF) et (iii) l'ajout audit sel en solution, d'un complexe à base de trioxyde de soufre tel que SO₃-pyridine, SO₃-triéthylamine ou SO₃-DMF en solution dans le même solvant, à une température inférieure à 70°C, le rapport molaire complexe à base de trioxyde de soufre/OH libres étant compris entre 0,25 et 12.

Les dérivés de dextrane obtenus conformément à un tel procédé, qui sont utilisés dans la composition pharmaceutique selon l'invention, présentent une homogénéité de la distribution des tailles de chaînes, illustrée par un profil d'élution de type gaussien symétrique en chromatographie d'exclusion stérique haute performance, et une homogénéité de la distribution des groupements chimiques chargés, illustrée par un profil d'élution à un seul pic symétrique en chromatographie d'échange d'ions basse pression. Ces dérivés de dextrane sont tels que la distribution des groupements chimiques confère au produit final une propriété biologique spécifique ; une telle distribution a pour conséquence que la composition chimique de chaque chaîne polysaccharidique est identique à la composition chimique globale du produit. De ce fait, il existe une composition chimique optimale pour une activité biologique spécifique maximale ; il y a donc une relation directe entre la propriété biologique considérée et la composition chimique globale du produit.

De façon particulièrement avantageuse, la composition selon l'invention, qui comprend au moins un dérivé de dextrane tel que décrit ci-dessus, à la dose unitaire sus-mentionnée, permet d'obtenir une action cicatrisante particulièrement efficace.

Cette propriété est liée à l'interaction entre les dérivés de dextrane et les facteurs de croissance qui sont naturellement sécrétés au niveau d'un site lésé, comme cela a été démontré notamment par A. Meddahi *et al.* dans Journal of Biomedical Materials Research, 1996, 31, 293-297, par J. Lafont *et al.* dans Growth factors, 1998, 16, 23-38 et par F. Blanquaert *et al.* dans Journal of Biomedical Materials Research, 1999, 44, 63-72. F. Blanquaert *et al.* (*ibid*) ont montré qu'un degré de substitution élevé en unités sulfonates est important pour qu'un dérivé de dextrane interagisse avec des facteurs de croissance. De façon surprenante, les dérivés de dextrane utilisés dans la composition pharmaceutique selon l'invention, qui ne comprennent pas de groupes sulfonates, sont néanmoins capables de protéger les facteurs de croissance, tels que les FGFs (*Fibroblast Growth Factors*), les TGF-α et β (*Transforming Growth Factors*), les IGFs (*Insulin-like Growth Factors*), les EGFs (*Epidermal Growth factors*) et les PDGFs (*Platelet-Derived Growth factors*) contre les dégradations protéolytiques et de favoriser ainsi leur action bénéfique sur la cicatrisation.

L'invention a également pour objet l'utilisation de la composition pharmaceutique définie ci-dessus pour la préparation d'un médicament à action cicatrisante.

Notamment, l'invention a pour objet l'utilisation de la composition pharmaceutique définie ci-dessus pour la préparation d'un médicament à action sur la cicatrisation de la muqueuse gastrique, auquel cas le dérivé de dextrane est de préférence présent, dans la composition pharmaceutique, à une dose unitaire comprise entre 1,5 et 10 mg.

Dans cette utilisation, la composition pharmaceutique, qui se présente avantageusement sous la forme d'un gel, d'un pansement gastrique, d'un sirop ou d'une solution buvable, est adaptée à une administration par voie orale.

De façon particulièrement avantageuse, le dérivé de dextrane peut être enrobé dans un vecteur permettant sa libération prolongée ou sa libération spécifique en un site donné. Il peut s'agir par exemple d'un enrobage gastrorésistant. L'invention a également pour objet l'utilisation de la composition pharmaceutique définie ci-dessus pour la préparation d'un médicament à action sur la cicatrisation musculaire, auquel cas le dérivé de dextrane est de préférence présent, dans la composition pharmaceutique, à une dose unitaire comprise entre 0,5 et 50 mg.

Une telle composition pharmaceutique se présente par exemple sous la forme d'un gel ou d'une pommade (pour une utilisation par application externe locale), ou encore sous la forme d'une solution isotonique, c'est-à-dire d'une solution dont la pression osmotique est la même que celle du sang. Dans ce dernier cas, la composition pharmaceutique est adaptée à une administration par voie parentérale, par exemple sous la forme d'une injection intramusculaire. L'invention a également pour objet l'utilisation de la composition pharmaceutique définie ci-dessus pour la préparation d'un médicament à action sur la cicatrisation oculaire, auquel cas le dérivé de dextrane est de préférence présent, dans la composition pharmaceutique, à une dose unitaire comprise entre 0,1 et 10 mg.

Dans cette utilisation, la composition pharmaceutique se présente par exemple sous la forme d'un collyre ou d'une pommade ophtalmique.

La présente invention a également pour objet une composition pharmaceutique à action sur la cicatrisation cutanée, adaptée à une administration par la voie topique, comprenant :
**(1)** au moins un dérivé de dextrane de formule générale DMCₐB_{b}Su_{c} tel que défini précédemment,
**(2)** ainsi qu'au moins un excipient acceptable du point de vue pharmaceutique,
ledit dérivé de dextrane étant présent à une concentration inférieure à 10 % (en poids/volume).

L'invention a également pour objet l'utilisation d'une telle composition pharmaceutique pour la préparation d'un médicament à action sur la cicatrisation cutanée, destiné à être administré par la voie topique.

Dans cette utilisation, la composition pharmaceutique peut se présenter sous la forme d'une pâte, d'une pommade, d'un liquide aqueux, d'un liquide huileux, d'un gel aqueux, d'un gel huileux, d'un aérosol, d'une mousse, d'une microémulsion, d'une émulsion multiple, de liposomes ou de nanoparticules.

On entend par « pâte » une pâte anhydre, par exemple à base de propylène glycol, de glycérol ou d'acide stéarique. Une pommade peut être obtenue en utilisant du polyéthylène glycol, de la vaseline ou encore de la paraffine liquide.

Ladite composition pharmaceutique pourrait également, par exemple, se présenter sous la forme d'une poudre, c'est-à-dire d'un lyophilisat apte à être remis sous la forme d'une solution au moment de son utilisation.

La composition pharmaceutique à action sur la cicatrisation cutanée selon l'invention peut être appliquée sur la plaie cutanée par la voie topique (voie locale externe), soit directement, soit par l'intermédiaire d'un dispositif médical tel qu'une compresse, de façon classique une compresse en coton ou en tissu, qui est imbibée de la composition selon l'invention, à la concentration indiquée ci-dessus.

La présente invention a également pour objet une composition pharmaceutique à action anti-complémentaire comprenant :
**(1)** au moins un dérivé de dextrane de formule générale DMCₐB_{b}Su_{c}, dans laquelle :
   D représente une chaîne polysaccharidique, de préférence constituée par des enchaînements d'unités glucosidiques,
   MC représente des groupes méthylcarboxylates,
   B représente des groupes carboxyméthylbenzylamides,
   Su représente des groupes sulfates (sulfatation des fonctions hydroxyles libres portées par les unités glucosidiques),
   a, b et c représentent le degré de substitution (ds), exprimé par rapport au nombre de fonctions hydroxyles libres dans une unité glucosidique du dextrane, respectivement en groupements MC, B et Su ; a étant ≥ à 0,3, b étant ≥ à 0,1 et c étant compris entre 0,1 et 0,4,
**(2)** ainsi qu'au moins un excipient acceptable du point de vue pharmaceutique,
ledit dérivé de dextrane étant présent à une dose unitaire comprise entre 5 et 30 mg.

On entend par « excipient » tout adjuvant ou véhicule sans action pharmacologique mais permettant la fabrication, la conservation ou l'administration de la composition pharmaceutique. Tout excipient acceptable du point de vue pharmaceutique, choisi par exemple parmi les excipients couramment utilisés en galénique, peut être utilisé dans la composition pharmaceutique à action anti-complémentaire selon l'invention.

La composition pharmaceutique à action anti-complémentaire selon l'invention comprend ainsi des dérivés de dextrane qui sont significativement différents de ceux décrits dans l'Art antérieur sous les dénominations RGTA9 et 11, et ce notamment en ce qu'ils ne comprennent pas d'unités sulfonates.

Les dérivés de dextrane de formule générale DMCₐB_{b}Su_{c} présents dans la composition pharmaceutique à action anti-complémentaire selon l'invention sont identiques à ceux décrits précédemment en rapport avec les compositions pharmaceutiques à action cicatrisante conformes à la présente invention. Ils peuvent notamment être obtenus par le procédé décrit dans le Brevet français 2 772 382, auquel cas ils présentent une homogénéité de la distribution des tailles de chaînes, illustrée par un profil d'élution de type gaussien symétrique en chromatographie d'exclusion stérique haute performance, et une homogénéité de la distribution des groupements chimiques chargés, illustrée par un profil d'élution à un seul pic symétrique en chromatographie d'échange d'ions basse pression.

De façon particulièrement avantageuse, la composition pharmaceutique selon l'invention, qui comprend au moins un dérivé de dextrane tel que décrit ci-dessus, à la dose unitaire sus-mentionnée, permet d'obtenir une action anti-complémentaire particulièrement efficace, et peut être utilisée dans tous les types de maladies ou de traitements faisant intervenir l'activation du système du complément (maladies autoimmunes, rejets de greffes, épurations plasmatiques ou sanguines ...).

L'invention a également pour objet l'utilisation de la composition pharmaceutique à action anti-complémentaire définie ci-dessus pour la préparation d'un médicament à action anti-complémentaire.

Dans cette utilisation, la composition pharmaceutique se présente avantageusement sous la forme d'une solution isotonique. Elle est alors administrée par injection (par exemple une injection intraveineuse ou intramusculaire).

La présente invention a, en outre, pour objet un pansement, caractérisé en ce qu'il est imbibé de la composition pharmaceutique à action sur la cicatrisation cutanée, adaptée à une administration par la voie topique, telle que décrite précédemment.

On ne sortirait pas du cadre de la présente invention en ajoutant aux compositions pharmaceutiques à action cicatrisante ou anti-complémentaire décrites ci-dessus, si cela est nécessaire, des additifs acceptables du point de vue pharmaceutiques, par exemple des conservateurs, des antioxydants, des antibactériens, des facteurs de pénétration, des colorants, des édulcorants et des arômes, ainsi que un ou plusieurs autres principes actifs, par exemple un antibiotique.

Les compositions pharmaceutiques à action cicatrisante ou anti-complémentaire conformes à l'invention peuvent être utilisées aussi bien en santé humaine qu'en santé animale (c'est-à-dire dans le cadre d'un usage vétérinaire).

En ce qui concerne les doses unitaires mentionnées ci-dessus pour les compositions pharmaceutiques à activité cicatrisante ou anti-complémentaire selon l'invention, elles sont indiquées par rapport à un individu adulte d'environ 70 kg ; toutefois, il est bien entendu que l'Homme de l'Art adaptera ces doses en fonction du poids, de l'âge et de la pathologie ou des symptômes de l'individu.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

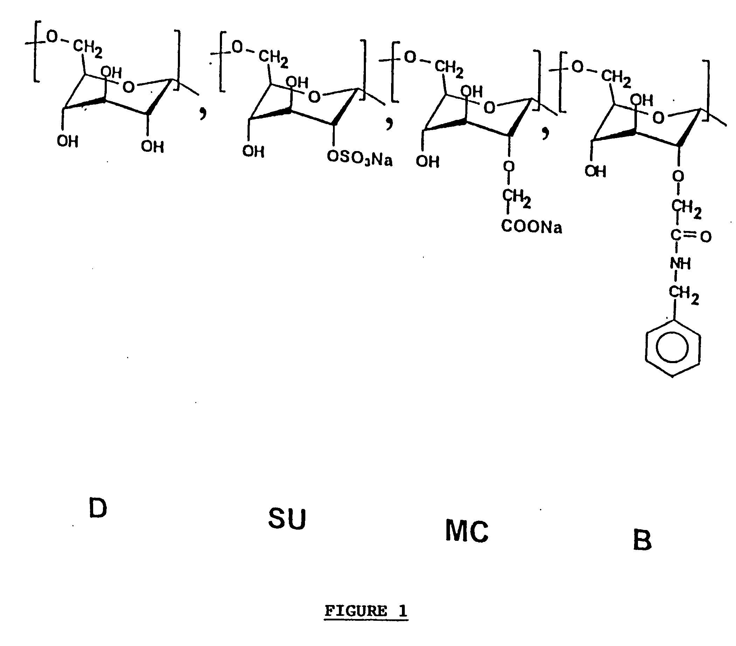

- la figure 1 illustre schématiquement la structure d'un dextrane substitué par les différents groupements chimiques fixés sur les unités glucosidiques ; la position du substituant sur les différents carbones des unités de base glucosidiques est présentée en 2, à titre d'exemple ;
- la figure 2 illustre l'activité anti-complémentaire d'un dérivé de dextrane de formule générale DMCₐB_{b}Su_{c} ; dans cette figure, le CH50 (%), mesuré comme indiqué dans l'exemple 5, est représenté en fonction du temps (heures) ;
- la figure 3 représente la cicatrisation, après 3 et 7 jours (J3 et J7), d'incisions cutanées dorsales pratiquées sur des rats, les plaies étant traitées soit par une solution physiologique (photographies de la colonne 1), soit par une solution d'un dérivé de dextrane de formule générale DMCₐB_{b}Su_{c} (photographies de la colonne 2).

Il doit être bien entendu, toutefois, que ces exemples sont donnés uniquement à titre d'illustration de l'objet de l'invention.

### EXEMPLE 1 : Absence de groupes sulfonates dans les différents dérivés de dextrane de formule générale DMCₐB_{b}Su_{c} utilisés dans les compositions pharmaceutiques selon la présente invention.

### a) Protocole

Le protocole de désulfatation suivant, propre à désulfater divers produits sans éliminer des groupes sulfonates éventuellement présents, a été suivi.

Le produit sous forme de sel de sodium (250 mg, 10 ml) est agité lentement à température ambiante avec 3 ml de résine échangeuse de cations (Amberlite^{®} IR120 H+, 16-45 mesh, capacité d'échange totale : 1,9 meq/ml). Après 2 h, la solution acide est filtrée, neutralisée avec de la pyridine (1 à 2 ml) jusqu'à un pH de 6-6,5 et évaporée à sec. Le sel de pyridinium obtenu est repris 3 fois avec 10 ml de méthanol anhydre et évaporé à sec.

Le résidu est dispersé dans 25 ml d'un mélange 90:9:1 en diméthylsulfoxyde (DMSO), méthanol et pyridine. La solution est agitée dans un bain d'huile chauffé à 90°C pendant 72 h. La réaction est arrêtée en ajoutant 20 ml d'eau bidistillée froide, puis le mélange est neutralisé avec une solution aqueuse de NaOH 1M. Le produit désulfaté est purifié par chromatographie d'exclusion stérique basse pression sur une colonne Séphadex^{®} G15 puis diafiltré sur cellule équipée d'une membrane de seuil de coupure 1000 Da. Entre 160 et 210 mg de produit désulfaté sont obtenus.

Les produits soumis à ce protocole sont des dérivés de dextrane dont les compositions sont les suivantes, D, MC, B, Su et S représentant respectivement les unités glucosidiques de la chaîne polysaccharidique, les groupes méthylcarboxylates, les groupes carboxyméthylbenzylamides, les groupes sulfates et les groupes sulfonates :
A: DMCₐB_{b}Su_{c}S_{d} : a = 0,87, b = 0,16, c = 0,5 et d = 0,10,
B: DMCₐB_{b}Su_{c}S_{d} : a = 0,87, b = 0,16, c = 0,6 et d = 0,07,
C : DMCₐB_{b}Su_{c}S_{d} : a = 0,87, b = 0,16, c = 1,0 et d = 0,05,
D : DMCₐB_{b}Su_{c} : a = 0,81, b = 0,18 et c = 0,40,
E: DMCₐB_{b}Su_{c} : a = 0,81, b = 0,18 et c = 0,30,
F : DMCₐSu_{c} : a = 0,95 et c = 0,48,
G : DMCₐSu_{c} : a = 0,95 et c = 0,93

Les composés A à C, F et G correspondent à des composés de référence, alors que les composés D et E correspondent à des dérivés de dextrane utilisés dans les compositions pharmaceutiques selon l'invention.

### b) Résultats

Le tableau 1 regroupe les teneurs en soufre, mesurées par analyse élémentaire, par rapport à 100 g du dérivé de dextrane, avant et après désulfatation pour chacun des produits A à G décrits ci-dessus.

**Tableau I : teneurs en soufre.**

| Dérivé de dextrane | Avant désulfatation. | Après désulfatation. |
|---|---|---|
| | Soufre (g/100 g) | Soufre (g/100 g) |
| A | 1,91 | 0,43 |
| B | 2,00 | 0,25 |
| C | 3,00 | 0,15 |
| D | 1,18 | 0 |
| E | 0,96 | 0 |
| F | 1,66 | 0 |
| G | 2,80 | 0 |

Après désulfatation, on constate l'absence totale de soufre dans les produits D à G, préparés à partir d'un complexe SO₃-pyridine, alors que les produits A à C présentent un taux en soufre nettement inférieur, mais non nul. Il ressort donc de ces résultats que l'absence de soufre correspond à l'absence de groupes sulfonates.

Pour confirmer ce résultat, un sel de sodium de l'acide sulfanilique (sel de sodium de l'acide para-aniline-sulfonique : H₂N-C₆H₅-SO₃Na) a été couplé à un dérivé de dextrane de formule générale DMCₐ, dans laquelle a = 0,95, en suivant le même procédé que celui précédemment décrit pour coupler la benzylamine sur un dérivé de dextrane portant des groupes carboxyméthyles. Le dérivé obtenu ne contient donc, outre des groupes carboxyméthyles, que des groupes sulfonates. Sa teneur en soufre est de 1,20 g/100 g. Après soumission de ce dérivé au protocole de désulfatation décrit ci-dessus, sa teneur en soufre est de 1,08 g/100 g. Les groupements sulfonates ne sont donc globalement pas atteints par le procédé de désulfatation. L'absence de soufre dans les dérivés du dextrane traités par désulfatation signifie donc bien une absence de groupes sulfonates.

Il ressort ainsi de cet exemple que les dérivés de dextrane de formule générale DMCₐB_{b}Su_{c} utilisés dans les compositions pharmaceutiques selon la présente invention ne possèdent pas de groupes sulfonates.

### EXEMPLE 2 : Action d'un dérivé de dextrane de formule générale DMCₐB_{b}Su_{c} in vivo sur la cicatrisation cutanée.

### a) Protocole

Les animaux utilisés sont des lapins albinos de souche néo-zélandaise, âgés de 1 an et pesant de 3,5 à 4,0 kg. On utilise deux lots d'animaux, chacun étant constitué de 6 mâles et de 6 femelles. Le premier lot est traité par un dérivé de dextrane de formule générale DMCₐB_{b}Su_{c} avec a = 0,80, b = 0,20 et c = 0,20, alors que le deuxième lot sert de contrôle, les animaux ne recevant qu'une solution physiologique.

Le protocole suivi est le suivant :
- deux plaies cutanées dermo-épidermiques de 6 mm de diamètre sont pratiquées de part et d'autre de l'axe vertébral,
- une compresse imbibée, selon les lots d'animaux, soit d'une solution à 50 µg/ml, dans un tampon PBS, du dérivé de dextrane mentionné ci-dessus, soit d'une solution physiologique, est appliquée deux fois par jour sur les plaies cutanées,
- des examens des plaies et de l'état général des animaux sont réalisés quotidiennement, l'évolution de la cicatrisation étant effectuée par des prélèvements au niveau des plaies à J2, J4, J6, J8, J10, J15 et J30 (le processus cicatriciel est considéré en jours à partir de la date à laquelle les plaies cutanées sont effectuées),
- les prélèvements sont préparés sous forme de coupes et sont analysés par des études histologiques.

### b) Résultats

Les différents phénomènes histologiques constatés sont résumés dans les tableaux II à IV ci-après.

Le tableau II résume l'état de la matrice extracellulaire en fonction du nombre de jours de cicatrisation, selon les critères suivants : présence de cellules inflammatoires et de fibroblastes (0 : absence de ces cellules ; +, + + et + + + : présence de ces cellules, leur nombre étant d'autant plus important que le nombre de signes + est élevé) et caractéristiques des vaisseaux sanguins (+ : présence de vaisseaux ; 0 : absence de vaisseaux ; haut : vaisseaux étudiés sur la partie supérieure de la coupe histologique ; bas : vaisseaux étudiés sur la partie inférieure de la coupe histologique, correspondant à la partie la plus proche du derme).

**Tableau II : matrice extracellulaire.**

| **Jours de cicatrisation** | **Cellules inflammatoires** | | **Fibroblastes** | | **Vaisseaux (vx)** | |
|---|---|---|---|---|---|---|
| | **Animaux traités** | **contrôle** | **Animaux traités** | **contrôle** | **Animaux traités** | **contrôle** |
| 2 | + | + + + | + | 0 | 0 | 0 |
| 4 | + | + + | + + | + | haut : vx | haut :0 |
| | | | | | arrondis | bas : vx |
| | | | | | bas : vx | dirigés vers le haut de la plaie |
| | | | | | matures | |
| 6 | 0 | + + | + + + | + + | vx matures | haut : vx |
| | | | | | | allongés et matures |
| 15 | 0 | 0 | + | ++ | + | + |
| 30 | 0 | 0 | + | + | peu de vx | peu de vx |

Il ressort du tableau II que la maturation de la matrice extracellulaire est plus importante et plus précoce chez les animaux traités par le dérivé de dextrane par rapport au groupe de contrôle. En particulier, les cellules inflammatoires sont moins nombreuses dans les plaies traitées par le dérivé de dextrane, par rapport aux plaies non, traitées, qui révèlent une persistance des phénomènes inflammatoires. Dans les plaies traitées par le dérivé de dextrane, on note également que les fibroblastes, outre leur apparition plus précoce, sont orientés et produisent une matrice extracellulaire plus importante.

Le tableau III résume l'état de l'épiderme, dans les cas où il est présent, en fonction du nombre de jours de cicatrisation, le terme « migration » indiquant une initiation de la reconstruction de l'épiderme par la migration des kératinocytes.

**Tableau III : épiderme.**

| jours de cicatrisation | animaux traités | contrôle |
|---|---|---|
| 2 | absence | absence |
| 4 | migration | migration |
| 6 | reconstitué et différencié | migration |
| 15 | mature | reconstitué et en maturation |
| 30 | mature | mature |

De façon classique, la régénération de l'épiderme implique une migration des cellules du derme, suivie d'une phase de reconstruction, au cours de laquelle les cellules prolifèrent, puis d'une maturation de l'épiderme régénéré. Il ressort du tableau III que l'épiderme se reconstruit nettement plus vite chez les animaux traités par le dérivé de dextrane que chez les groupe de contrôle.

Le tableau IV ci-dessous résume la cinétique d'apparition du facteur de croissance TGFβ (*Transforming Growth Factor*) au niveau des plaies en fonction du nombre de jours de cicatrisation (0 : absence de TGFβ ; +, + + et + + + : présence de TGFβ, en quantité d'autant plus importante que le nombre de signes + est élevé).

**Tableau IV : cinétique d'apparition du facteur de croissance TGFβ.**

| jours de cicatrisation | animaux traités | contrôle |
|---|---|---|
| 2 | 0 | 0 |
| 4 | + + + | + |
| 6 | + | + + |
| 15 | 0 | 0 |
| 30 | 0 | 0 |

Il ressort du tableau IV que la cinétique d'apparition du facteur de croissance TGFβ diffère chez les animaux traités par un dérivé de dextrane par rapport aux animaux non traités : le TGFβ apparaît en grande quantité dès J4 chez les animaux traités, alors qu'il n'apparaît substantiellement qu'à J6 chez les animaux non traités, et ce en quantité moindre.

### EXEMPLE 3 : Autre protocole d'évaluation de l'action in vivo d'un dérivé de dextrane de formule générale DMCₐB_{b}Su_{c} sur la cicatrisation cutanée.

### a) Protocole.

Les animaux utilisés sont des rats « hairless » âgés de 13 semaines, sur lesquels sont pratiquées des incisions cutanées dorsales de 6 cm de longueur. Quatre points de suture sous-cutanés au fil tressé résorbable (polyglatine 910, Vicryl^{®}, taille 5/0, ETHICON, France) espacés les uns des autres de 1,5 cm sont réalisés afin de permettre le rapprochement et l'apposition des berges de l'incision.

Les animaux sont répartis en deux groupes:
- un premier groupe de 10 rats est traité par une solution à 50 µg/ml, dans un tampon PBS, d'un dérivé de dextrane de formule générale DMCₐB_{b}Su_{c} avec a = 0,65, b = 0,12 et c = 0,33,
- un second groupe de 10 rats, servant de témoin négatif, est traité uniquement par une solution physiologique.

Les plaies sont traitées une fois par jour pendant 4 jours avec les solutions mentionnées ci-dessus, par application topique à l'aide d'une compresse imbibée desdites solutions. Deux heures après avoir été traitées, les plaies sont recouvertes d'un pansement stérile. Une étude de résistance à la traction des cicatrices est réalisée après 3 et 7 jours (J3 et J7) à partir de bandelettes cutanées prélevées au niveau de la plaie. Un tensiomètre est utilisé pour mesurer la traction maximale (en grammes) exercée sur ces échantillons cutanés avant rupture.

### b) Résultats.

La résistance à la rupture des plaies à J3 et à J7 est la suivante :
- plaies traitées par le dérivé de dextrane : 1500 grammes (J3) et 2200 grammes (J7),
- plaies traitées uniquement par une solution physiologique : 900 grammes (J3) et 1200 grammes (J7),

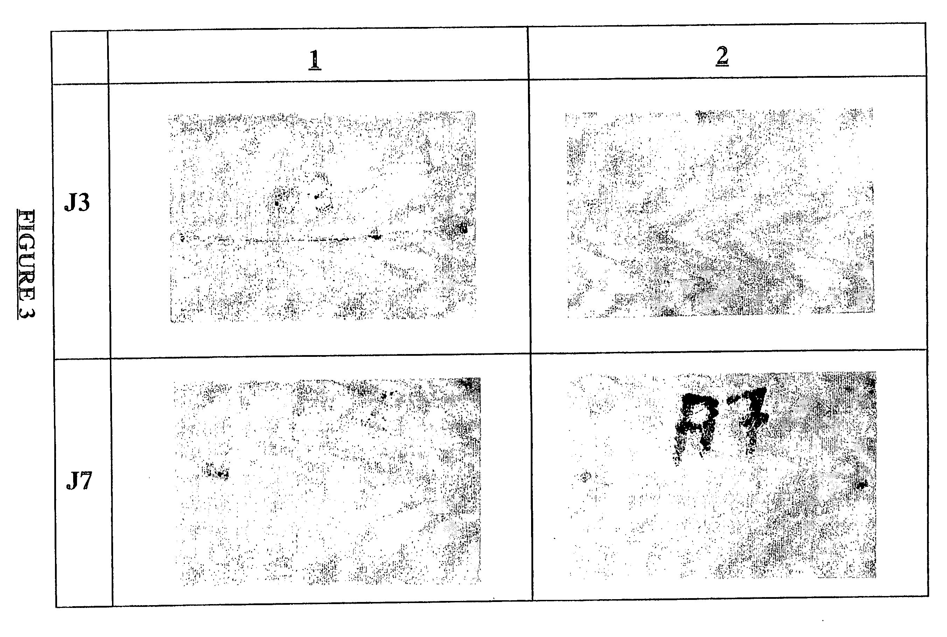

La figure 3 représente des photographies des plaies traitées uniquement par une solution physiologique (photographies de la colonne 1) et des plaies traitées par la solution du dérivé de dextrane (photographies de la colonne 2). On remarque une cicatrisation de bien meilleure qualité lorsque les plaies sont traitées à l'aide du dérivé de dextrane, par rapport aux plaies des animaux témoins.

### EXEMPLE 4 : Action d'un dérivé de dextrane de formule générale DMCₐB_{b}Su_{c} in vivo sur la cicatrisation gastrique.

### a) Protocole

Les animaux utilisés sont des rats mâles Sprague-Dawley, âgés de 12 semaines et pesant de 200 à 250 g. On utilise trois lots d'animaux, chacun étant constitué de 4 animaux :
- le premier lot est traité par un dérivé de dextrane de formule générale DMCₐB_{b}Su_{c} avec a = 0,75, b = 0,25 et c = 0,15, à raison de 50 µg/kg,
- le deuxième lot sert de contrôle, les animaux étant traités par un dextrane natif T40 (Pharmacia Fine Chemical ; masse molaire moyenne en poids M_{w} : 37500 g/mol ; M_{w}/Mₙ : 1,7, Mₙ représentant la masse moyenne en nombre), à raison de 50 µg/kg,
- le troisième lot est traité par la prostaglandine E2 (Sigma ; dose de 10 µg/kg), qui a un effet protecteur de la muqueuse gastrique contre les produits irritants induisant une réaction inflammatoire locale. Ce lot correspond donc à un témoin positif.

Le dérivé de dextrane, le dextrane natif et la prostaglandine E2 sont dissous dans du sérum physiologique et administrés aux animaux par voie orale.

Les animaux sont sacrifiés à différents stades de la cicatrisation, à savoir à J2, J4, J7 et J14, le processus cicatriciel étant considéré en jours à partir de la date à laquelle l'irritation gastrique est induite. La muqueuse gastrique est récupérée en vue de deux études :
- la quantification de l'atteinte de la muqueuse gastrique,
- l'isolement des récepteurs de facteurs de croissance (EGF : *Epidermal Growth Factor* ; PDGF : *Platelet-Derived Growth Factor* et TGF : *Transforming Growth Factor*). Ces facteurs de croissance sont en effet connus pour leur rôle important dans la cicatrisation gastrique : le TGF et l'EGF contrôlent la prolifération cellulaire en se fixant sur leurs récepteurs, alors que l'EGF favorise la restauration tissulaire et l'apparition de microvaisseaux.

### b) Résultats

### • Atteinte de la muqueuse gastrique.

Le tableau V regroupe les résultats de l'étude macroscopique de la réaction inflammatoire au niveau de la muqueuse gastrique, effectuée selon le barème suivant :
(0) Muqueuse normale.
(+) Petites stries hémorragiques superficielles.
(+ +) Stries hémorragiques superficielles, courtes et larges.
(+ + +) Stries hémorragiques superficielles, longues et larges.
(+ + + +) Perforations.

**Tableau V : atteinte de la muqueuse gastrique.**

| jours | dérivé de dextrane | prostaglandine E2 | dextrane T40 |
|---|---|---|---|
| J2 | + + | + + | + + + |
| J4 | + | + | + + + |
| J7 | + | + | + + |
| J14 | 0 | 0 | + + |

Il ressort du tableau V que le dérivé de dextrane exerce des effets comparables à ceux de la prostaglandine E2, mais bien meilleurs que ceux du dextrane T40.

Ces résultats traduisent le fait que le dérivé de dextrane est capable de protéger les facteurs de croissance endogènes responsables de l'accélération de la cicatrisation de la muqueuse gastrique.

### • Récepteurs de facteurs de croissance.

La cicatrisation gastrique se manifeste par l'augmentation des récepteurs de deux facteurs de croissance : EGF et PDGF. Au jour J4, le groupe traité par le dérivé de dextrane présente 1,7 à 1,8 fois plus de récepteurs à ces deux facteurs de croissance que le groupe traité par le dextrane T40. Au jour J7, la quantité de récepteurs à ces deux facteurs de croissance est 3 fois supérieure dans le groupe traité par le dérivé de dextrane, par rapport au groupe traité par le dextrane T40.

### EXEMPLE 5 : Action d'un dérivé de dextrane de formule générale DMCₐB_{b}Su_{c} in vivo sur la cicatrisation musculaire.

### a) Protocole

Les animaux utilisés sont des rats mâles Wistar, âgés de 12 semaines et pesant de 200 à 300 g. On utilise deux lots d'animaux, chacun étant constitué de 6 rats.

Le protocole suivi est le suivant : après anesthésie par de l'éther, les muscles des pattes postérieures sont dégagés de la loge antérieure de la jambe et lésés mécaniquement par application, pendant 10 secondes, d'une pression constante sur toute la longueur du muscle à l'aide d'une pince de péan maintenue au deuxième cran.

Le muscle est ensuite replacé dans sa loge après avoir reçu une injection de 200 µl soit d'une solution de 20 µg/ml de dérivé de dextrane de formule générale DMCₐB_{b}Su_{c} avec a = 0,75, b = 0,25 et c = 0,15 (patte droite), soit d'une solution physiologique (patte gauche). Le muscle est laissé en place pendant 3 minutes afin de laisser le produit diffuser, puis la peau est suturée.

Les muscles traités (pattes postérieures droites et gauches) sont prélevés après 7 jours et congelés à -150°C. Des coupes transversales de 10 µm sont alors effectuées dans la région médiane du muscle. Les coupes séchées sont colorées par le trichrome de Gomori.

### b) Résultats

Le tableau VI regroupe l'analyse morphométrique du nombre de fibres et de leur diamètre, effectuée sur des montages micrographiques correspondant à une hémi-coupe transversale du muscle.

**Tableau VI : Analyse morphométrique des muscles.**

| | Muscle traité par le dérivé de dextrane | Muscle traité par le sérum physiologique |
|---|---|---|
| Diamètre des muscles (mm) | 6,5 ± 0,3 | 4,3 ± 0,2 |
| Nombre de faisceaux musculaires par coupe | 21 ± 2 | 11 ± 3 |
| Nombre de fibres par faisceau | 78 ± 10 | 65 ± 5 |
| Densité des fibres (nombre/mm²) | 722 ± 52 | 83 ± 12 |

Il ressort du tableau VI que les muscles traités par le dérivé de dextrane se régénèrent plus rapidement que les muscles non traités : la densité de fibres est nettement plus élevée que celle des muscles n'ayant reçu qu'une solution de sérum physiologique. Le nombre de faisceaux musculaires et le diamètre des muscles traduisent respectivement une réorganisation musculaire accélérée et un degré de maturation des muscles amélioré dans le cas d'un traitement par le dérivé de dextrane.

### EXEMPLE 6 : Action d'un dérivé de dextrane de formule générale DMCₐB_{b}Su_{c} in vivo sur la cicatrisation oculaire.

### a) Protocole

On utilise 20 lapins albinos de souche néo-zélandaise (10 mâles et 10 femelles), âgés de 1 an et pesant de 2,5 à 3 kg, auxquels un filtre de 5,5 mm imbibé d'une solution de soude IN a été placé pendant 60 secondes dans l'oeil droit, et qui sont répartis en 4 groupes de 5 animaux :
- le premier groupe est traité, trois fois par jour, par 100 µl d'une solution contenant 0,1 ng de FGF-2 (*Fibroblast Growth Factor-2*) ;
- le second groupe est traité, trois fois par jour, par 100 µl d'une solution isotonique contenant 50 µg/ml de dérivé de dextrane de formule générale DMCₐB_{b}Su_{c} avec a = 0,75, b = 0,31 et c = 0,34 ;
- le troisième groupe est traité, trois fois par jour, par 100 µl d'une solution contenant 0,1 ng de FGF-2 et 50 µg/ml du dérivé de dextrane sus-mentionné ;
- le quatrième groupe (contrôle) est traité, trois fois par jour, par 100 µl d'une solution de tampon PBS.

La vitesse de cicatrisation oculaire des quatre groupes d'animaux est comparée par des études histologiques réalisées aux jours J1, J2, J4, J6 et J8.

### b) Résultats

Il ressort des études histologiques réalisées que la durée de cicatrisation oculaire, exprimée selon une moyenne pour chaque groupe d'animaux, est de 4 jours pour le troisième groupe, 5 jours pour le premier groupe, 6 jours pour le second groupe et 8 jours pour le groupe de contrôle. La cicatrisation oculaire est donc plus rapide chez les animaux traités par le dérivé de dextrane, par rapport au groupe de contrôle. On remarque également que la cicatrisation est plus précoce quand le dérivé de dextrane est combiné à un facteur de croissance, et ce en comparaison au dérivé de dextrane et au facteur de croissance seul, ce qui montre l'effet protecteur et potentialisateur du dérivé de dextrane utilisé dans les compositions selon la présente invention vis-à-vis des facteurs de croissance.

### EXEMPLE 7 : Action anti-complémentaire in vivo d'un dérivé de dextrane de formule générale DMCₐB_{b}Su_{c}.

### a) Protocole

Les animaux utilisés sont des rats mâles Sprague-Dawley, âgés de 12 semaines et pesant de 200 à 300 g. On utilise trois lots d'animaux, chacun étant constitué de 5 rats :
- le premier lot reçoit une injection de 500 µl d'une solution contenant du Séphadex^{®} G25 à raison de 20 µg (le Séphadex^{®} est un activateur de la voie alterne du système du complément chez l'homme),
- le second lot reçoit une injection de 500 µl d'une solution contenant du Séphadex^{®} G25 à raison de 20 µg ainsi que 50 µg de dextrane natif (40 000 g/mol),
- le troisième groupe reçoit une injection de 500 µl d'une solution contenant du Séphadex^{®} G25 à raison de 20 µg ainsi que 50 µg de dérivé de dextrane de formule générale DMCₐB_{b}Su_{c} avec a = 0,60, b = 0,35 et c = 0,25.

En tant qu'activateur du système du complément, le lambda-carraghénane peut être utilisé à la place du Séphadex^{®} selon le protocole suivant, qui aboutit aux mêmes résultats que ceux indiqués ci-après :
- le premier lot d'animaux reçoit une injection de 200 µl d'une solution de lambda-carraghénane à 1% (m/V) dans du NaCl à 0,9%,
- le second lot d'animaux reçoit une injection de 200 µl de cette solution de de lambda-carraghénane, suivie (30 minutes après) d'une injection de 100 µl du dérivé de dextrane,
- le troisième lot d'animaux reçoit une injection de 200 µl de cette solution de de lambda-carraghénane, suivie (30 minutes après) d'une injection de 100 µl d'une solution saline à 0,9%.

Le clivage de la protéine C3 est déterminé par immunoélectrophorèse avec un anticorps anti-C3.

L'action anticomplémentaire est étudiée selon un dosage hémolytique ou CH50, selon un protocole dans lequel le sérum des rats, prélevé à différents intervalles de temps, est activé par des érythrocytes de mouton sensibilisés par des anticorps de lapin antiglobules rouges de mouton (EA). Par définition, une unité CH50 correspond à la concentration de protéines du complément (contenue dans un millilitre de sérum) capable d'induire l'hémolyse de 50 % de 2 x 10⁷ EA activés dans un milieu réactionnel où sont maintenus constants le volume, la température et le temps de réaction. Le nombre de sites hémolytiques par cellule est calculé.

### b) Résultats

Une immunoélectrophorèse avec un anticorps anti-C3 faite sur le sérum des rats ayant reçu le dérivé de dextrane (troisième lot d'animaux) ne montre pas de clivage de la protéine C3, contrairement aux sérums des rats ayant reçu le dextrane natif et/ou le Séphadex^{®} (premier et second lots d'animaux).

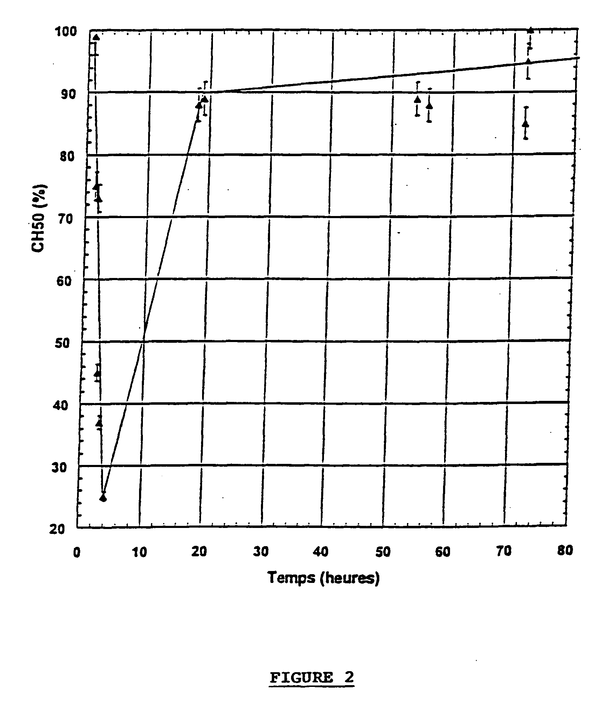

Le dextrane natif n'a aucun effet sur l'inhibition de l'activation du complément.
Le dérivé de dextrane utilisé dans la composition pharmaceutique selon l'invention induit une action inhibitrice du complément très rapide, comme le montre la figure 2, qui représente le CH50 (%) en fonction du temps (heures). Cette se poursuit 4 heures après l'injection. Le CH50 revient à son niveau initial 20 heures après l'injection.

## Revendications

1. Composition pharmaceutique à action cicatrisante comprenant :
**(1)** au moins un dérivé de dextrane de formule générale DMCₐB_{b}Su_{c}, dans laquelle :
D représente une chaîne polysaccharidique, de préférence constituée par des enchaînements d'unités glucosidiques,
MC représente des groupes méthylcarboxylates,
B représente des groupes carboxyméthylbenzylamides,
Su représente des groupes sulfates (sulfatation des fonctions hydroxyles libres portées par les unités glucosidiques),
a, b et c représentent le degré de substitution (ds), exprimé par rapport au nombre de fonctions hydroxyles libres dans une unité glucosidique du dextrane, respectivement en groupements MC, B et Su ; a étant ≥ à 0,6, b étant ≥ à 0,1 et c étant compris entre 0,1 et 0,5,
lesquels produits présentent une homogénéité de la distribution des tailles de chaînes, illustrée par un profil d'élution de type gaussien symétrique en chromatographie d'exclusion stérique haute performance, et une homogénéité de la distribution des groupements chimiques chargés, illustrée par un profil d'élution à un seul pic symétrique en chromatographie d'échange d'ions basse pression,
**(2)** ainsi qu'au moins un excipient acceptable du point de vue pharmaceutique,
ledit dérivé de dextrane étant présent à une dose unitaire comprise entre 0,1 et 50 mg.

2. Utilisation de la composition pharmaceutique selon la revendication 1 pour la préparation d'un médicament à action cicatrisante.

3. Utilisation de la composition pharmaceutique selon la revendication 1 pour la préparation d'un médicament à action sur la cicatrisation de la muqueuse gastrique.

4. Utilisation selon la revendication 3, **caractérisée en ce que** la dose unitaire dudit dérivé de dextrane est comprise entre 1,5 et 10 mg.

5. Utilisation selon la revendication 3 ou la revendication 4, **caractérisée en ce que** ladite composition pharmaceutique se présente sous la forme d'un gel, d'un pansement gastrique, d'un sirop ou d'une solution buvable.

6. Utilisation selon l'une quelconque des revendications 3 à 5, **caractérisée en ce que** ledit dérivé de dextrane est enrobé dans un vecteur.

7. Utilisation selon l'une quelconque des revendications 3 à 6, **caractérisée en ce que** ladite composition pharmaceutique est adaptée à une administration par voie orale.

8. Utilisation de la composition pharmaceutique selon la revendication 1 pour la préparation d'un médicament à action sur la cicatrisation musculaire.

9. Utilisation selon la revendication 8, **caractérisée en ce que** la dose unitaire dudit dérivé de dextrane est comprise entre 0,5 et 50 mg.

10. Utilisation selon la revendication 8 ou la revendication 9, **caractérisée en ce que** ladite composition pharmaceutique se présente sous la forme d'un gel, d'une pommade ou d'une solution isotonique.

11. Utilisation selon l'une quelconque des revendications 8 à 10, **caractérisée en ce que** ladite composition pharmaceutique est adaptée à une administration par application locale externe ou par voie parentérale.

12. Utilisation de la composition pharmaceutique selon la revendication 1 pour la préparation d'un médicament à action sur la cicatrisation oculaire.

13. Utilisation selon la revendication 12, **caractérisée en ce que** la dose unitaire dudit dérivé de dextrane est comprise entre 0,1 et 10 mg.

14. Utilisation selon la revendication 12 ou la revendication 13, **caractérisée en ce que** ladite composition pharmaceutique se présente sous la forme d'un collyre ou d'une pommade ophtalmique.

15. Composition pharmaceutique à action sur la cicatrisation cutanée, adaptée à une administration par la voie topique, comprenant :
**(1)** au moins un dérivé de dextrane de formule générale DMCₐB_{b}Su_{c}, dans laquelle :
D représente une chaîne polysaccharidique, de préférence constituée par des enchaînements d'unités glucosidiques,
MC représente des groupes méthylcarboxylates,
B représente des groupes carboxyméthylbenzylamides,
Su représente des groupes sulfates (sulfatation des fonctions hydroxyles libres portées par les unités glucosidiques),
a, b et c représentent le degré de substitution (ds), exprimé par rapport au nombre de fonctions hydroxyles libres dans une unité glucosidique du dextrane, respectivement en groupements MC, B et Su ; a étant ≥ à 0,6, b étant ≥ à 0,1 et c étant compris entre 0,1 et 0,5,
lesquels produits présentent une homogénéité de la distribution des tailles de chaînes, illustrée par un profil d'élution de type gaussien symétrique en chromatographie d'exclusion stérique haute performance, et une homogénéité de la distribution des groupements chimiques chargés, illustrée par un profil d'élution à un seul pic symétrique en chromatographie d'échange d'ions basse pression,
**(2)** ainsi qu'au moins un excipient acceptable du point de vue pharmaceutique,
ledit dérivé de dextrane étant présent à une concentration inférieure à 10 % (en poids/volume).

16. Utilisation de la composition pharmaceutique selon la revendication 15 pour la préparation d'un médicament à action sur la cicatrisation cutanée, destiné à être administré par la voie topique.

17. Utilisation selon la revendication 16, **caractérisée en ce que** ladite composition pharmaceutique se présente sous la forme d'une pâte, d'une pommade, d'un liquide aqueux, d'un liquide huileux, d'un gel aqueux, d'un gel huileux, d'un aérosol, d'une mousse, d'une microémulsion, d'une émulsion multiple, de liposomes ou de nanoparticules.

18. Composition pharmaceutique à action anti-complémentaire comprenant :
**(1)** au moins un dérivé de dextrane de formule générale DMCₐB_{b}Su_{c}, dans laquelle :
D représente une chaîne polysaccharidique, de préférence constituée par des enchaînements d'unités glucosidiques,
MC représente des groupes méthylcarboxylates,
B représente des groupes carboxyméthylbenzylamides,
Su représente des groupes sulfates (sulfatation des fonctions hydroxyles libres portées par les unités glucosidiques),
a, b et c représentent le degré de substitution (ds), exprimé par rapport au nombre de fonctions hydroxyles libres dans une unité glucosidique du dextrane, respectivement en groupements MC, B et Su; a étant ≥ à 0,3, b étant ≥ à 0,1 et c étant compris entre 0,1 et 0,4,
lesquels produits présentent une homogénéité de la distribution des tailles de chaînes, illustrée par un profil d'élution de type gaussien symétrique en chromatographie d'exclusion stérique haute performance, et une homogénéité de la distribution des groupements chimiques chargés, illustrée par un profil d'élution à un seul pic symétrique en chromatographie d'échange d'ions basse pression,
**(2)** ainsi qu'au moins un excipient acceptable du point de vue pharmaceutique,
ledit dérivé de dextrane étant présent à une dose unitaire comprise entre 5 et 30 mg.

19. Utilisation de la composition pharmaceutique selon la revendication 18 pour la préparation d'un médicament à action anti-complémentaire.

20. Utilisation selon la revendication 19, **caractérisée en ce que** ladite composition pharmaceutique se présente sous la forme d'une solution isotonique.

21. Pansement, **caractérisé en ce qu'**il est imbibé de la composition pharmaceutique selon la revendication 15.

## Claims

1. Pharmaceutical composition with wound healing activity, comprising:
**(1)** at least one dextran derivative of general formula DMCₐB_{b}Su_{c}, in which:
D represents a polysaccharide chain, preferably consisting of series of glucoside units,
MC represents methylcarboxylate groups,
B represents carboxymethylbenzylamide groups,
Su represents sulphate groups (sulphation of the free hydroxyl functions carried by the glucoside units),
a, b and c represent the degree of substitution (ds), expressed relative to the number of free hydroxyl functions in a glucoside unit of the dextran, respectively with respect to MC, B and Su groups; a being ≥ 0.6, b being ≥ 0.1 and c being between 0.1 and 0.5,
which products exhibit chain-size distribution homogeneity, illustrated by a symmetrical Gaussian elution profile in high performance steric exclusion chromatography, and charged-chemical-group distribution homogeneity, illustrated by an elution profile with a single symmetrical peak in low-pressure ion exchange chromatography,
**(2)** and also at least one pharmaceutically acceptable excipient,
said dextran derivative being present at a unit dose of between 0.1 and 50 mg.

2. Use of the pharmaceutical composition according to Claim 1, for preparing a medicament with wound healing activity.

3. Use of the pharmaceutical composition according to Claim 1, for preparing a medicament with activity on gastric mucosa wound healing.

4. Use according to Claim 3, **characterized in that** the unit dose of said dextran derivative is between 1.5 and 10 mg.

5. Use according to Claim 3 or Claim 4, **characterized in that** said pharmaceutical composition is in the form of a gel, a gastric dressing, a syrup or an oral solution.

6. Use according to any one of Claims 3 to 5, **characterized in that** said dextran derivative is coated in a vector.

7. Use according to any one of Claims 3 to 6, **characterized in that** said pharmaceutical composition is suitable for oral administration.

8. Use of the pharmaceutical composition according to Claim 1, for preparing a medicament with activity on muscle wound healing.

9. Use according to Claim 8, **characterized in that** the unit dose of said dextran derivative is between 0.5 and 50 mg.

10. Use according to Claim 8 or Claim 9, **characterized in that** said pharmaceutical composition is in the form of a gel, an ointment or an isotonic solution.

11. Use according to any one of Claims 8 to 10, **characterized in that** said pharmaceutical composition is suitable for administration by external local application or parenteral administration.

12. Use of the pharmaceutical composition according to Claim 1, for preparing a medicament with activity on ocular wound healing.

13. Use according to Claim 12, **characterized in that** the unit dose of said dextran derivative is between 0.1 and 10 mg.

14. Use according to Claim 12 or Claim 13, **characterized in that** said pharmaceutical composition is in the form of an eye lotion or an ophthalmic ointment.

15. Pharmaceutical composition with activity on skin wound healing, suitable for topical administration, comprising:
**(1)** at least one dextran derivative of general formula DMCₐB_{b}Su_{c}, in which:
D represents a polysaccharide chain, preferably consisting of series of glucoside units,
MC represents methylcarboxylate groups,
B represents carboxymethylbenzylamide groups,
Su represents sulphate groups (sulphation of the free hydroxyl functions carried by the glucoside units),
a, b and c represent the degree of substitution (ds), expressed relative to the number of free hydroxyl functions in a glucoside unit of the dextran, respectively with respect to MC, B and Su groups; a being ≥ 0.6, b being ≥ 0.1 and c being between 0.1 and 0.5,
which products exhibit chain-size distribution homogeneity, illustrated by a symmetrical Gaussian elution profile in high performance steric exclusion chromatography, and charged-chemical-group distribution homogeneity, illustrated by an elution profile with a single symmetrical peak in low-pressure ion exchange chromatography,
**(2)** and also at least one pharmaceutically acceptable excipient,
said dextran derivative being present at a concentration of less than 10% (by weight/volume).

16. Use of the pharmaceutical composition according to Claim 15, for preparing a medicament with activity on skin wound healing, intended to be administered topically.

17. Use according to Claim 16, **characterized in that** said pharmaceutical composition is in the form of a paste, an ointment, an aqueous liquid, an oily liquid, an aqueous gel, an oily gel, an aerosol, a foam, a microemulsion, a multiple emulsion, liposomes or nanoparticles.

18. Pharmaceutical composition with anti-complementary activity, comprising:
**(1)** at least one dextran derivative of general formula DMCₐB_{b}Su_{c}, in which:
D represents a polysaccharide chain, preferably consisting of series of glucoside units,
MC represents methylcarboxylate groups,
B represents carboxymethylbenzylamide groups,
Su represents sulphate groups (sulphation of the free hydroxyl functions carried by the glucoside units),
a, b and c represent the degree of substitution (ds), expressed relative to the number of free hydroxyl functions in a glucoside unit of the dextran, respectively with respect to MC, B and Su groups; a being ≥ 0.3, b being ≥ 0.1 and c being between 0.1 and 0.4,
which products exhibit chain-size distribution homogeneity, illustrated by a symmetrical Gaussian elution profile in high performance steric exclusion chromatography, and charged-chemical-group distribution homogeneity, illustrated by an elution profile with a single symmetrical peak in low-pressure ion exchange chromatography,
**(2)** and also at least one pharmaceutically acceptable excipient,
said dextran derivative being present at a unit dose of between 5 and 30 mg.

19. Use of the pharmaceutical composition according to Claim 18, for preparing a medicament with anti-complementary activity.

20. Use according to Claim 19, **characterized in that** said pharmaceutical composition is in the form of an isotonic solution.

21. Dressing, **characterized in that** it is impregnated with the pharmaceutical composition according to Claim 15.

## Patentansprüche

1. Pharmazeutische Zusammensetzung mit narbenbildender Wirkung, umfassend:
(1) mindestens ein Dextranderivat der allgemeinen Formel DMCₐB_{b}Su_{c}, worin:
D für eine Polysaccharidkette steht, die vorzugsweise aus Aneinanderreihungen von Glucosideinheiten besteht,
MC für Methylcarboxylatgruppen steht,
B für Carboxymethylbenzylamidgruppen steht,
Su für Sulfatgruppen steht (Sulfatisierung der freien Hydroxylfunktionen an den Glucosideinheiten),
a, b und c jeweils für den Grad der Substitution (Sg), ausgedrückt bezogen auf die Anzahl der freien Hydroxylfunktionen in einer Glucosideinheit des Dextrans, in den Gruppen MC, B und Su stehen; wobei a ≥ 0,6; b ≥ 0,1 und c zwischen 0,1 und 0,5 ist,
wobei diese Produkte eine homogene Verteilung der Kettenlängen, die sich durch ein symmetrisches gaussförmiges Elutionsprofil bei der sterischen Hochleistungsausschlusschromatographie zeigt, und eine homogene Verteilung der geladenen chemischen Gruppen aufweisen, die sich durch ein Elutionsprofil mit einem symmetrischen Maximum bei der Niederdruck-Ionenaustauschchromatographie zeigt,
(2) sowie mindestens einen pharmazeutisch annehmbaren Excipienten,
wobei das Dextranderivat in einer Einheitsdosis zwischen 0,1 und 50 mg vorliegt.

2. Verwendung der pharmazeutischen Zusammensetzung nach Anspruch 1 zur Herstellung eines Medikaments mit narbenbildender Wirkung.

3. Verwendung der pharmazeutischen Zusammensetzung nach Anspruch 1 zur Herstellung eines Medikaments mit einer Wirkung auf die Narbenbildung der Magenschleimhaut.

4. Verwendung nach Anspruch 3, **dadurch gekennzeichnet, dass** die Einheitsdosis des Dextranderivats zwischen 1,5 und 10 mg beträgt.

5. Verwendung nach Anspruch 3 oder Anspruch 4, **dadurch gekennzeichnet, dass** die pharmazeutische Zusammensetzung in Form eines Gels, eines Magenpflasters, eines Sirups oder einer trinkbaren Lösung vorliegt.

6. Verwendung nach einem der Ansprüche 3 bis 5, **dadurch gekennzeichnet, dass** das Dextranderivat mit einem Träger überzogen ist.

7. Verwendung nach einem der Ansprüche 3 bis 6, **dadurch gekennzeichnet, dass** die pharmazeutische Zusammensetzung an eine Verabreichung auf oralem Weg angepasst ist.

8. Verwendung der pharmazeutischen Zusammensetzung nach Anspruch 1 zur Herstellung eines Medikaments mit einer Wirkung auf die Narbenbildung im Muskel.

9. Verwendung nach Anspruch 8, **dadurch gekennzeichnet, dass** die Einheitsdosis des Dextranderivats zwischen 0,5 und 50 mg beträgt.

10. Verwendung nach Anspruch 8 oder Anspruch 9, **dadurch gekennzeichnet, dass** die pharmazeutische Zusammensetzung in Form eines Gels, einer Salbe oder einer isotonischen Lösung vorliegt.

11. Verwendung nach einem der Ansprüche 8 bis 10, **dadurch gekennzeichnet, dass** die pharmazeutische Zusammensetzung an eine Verabreichung durch äußere lokale Applikation oder auf parenteralem Weg angepasst ist.

12. Verwendung der pharmazeutischen Zusammensetzung nach Anspruch 1 zur Herstellung eines Medikaments mit einer Wirkung auf die Narbenbildung im Auge.

13. Verwendung nach Anspruch 12, **dadurch gekennzeichnet, dass** die Einheitsdosis des Dextranderivats zwischen 0,1 und 10 mg beträgt.

14. Verwendung nach Anspruch 12 oder Anspruch 13, **dadurch gekennzeichnet, dass** die pharmazeutische Zusammensetzung in Form von Augentropfen oder einer ophthalmischen Salbe vorliegt.

15. Pharmazeutische Zusammensetzung mit einer Wirkung auf die Narbenbildung der Haut, die an eine Verabreichung auf topischem Weg angepasst ist, umfassend:
(1) mindestens ein Dextranderivat der allgemeinen Formel DMCₐB_{b}Su_{c}, worin:
D für eine Polysaccharidkette steht, die vorzugsweise aus Aneinanderreihungen von Glucosideinheiten besteht,
MC für Methylcarboxylatgruppen steht,
B für Carboxymethylbenzylamidgruppen steht,
Su für Sulfatgruppen steht (Sulfatisierung der freien Hydroxylfunktionen an den Glucosideinheiten),
a, b und c jeweils für den Grad der Substitution (Sg), ausgedrückt bezogen auf die Anzahl der freien Hydroxylfunktionen in einer Glucosideinheit des Dextrans, in den Gruppen MC, B und Su stehen; wobei a ≥ 0,6; b ≥ 0,1 und c zwischen 0,1 und 0,5 ist,
wobei diese Produkte eine homogene Verteilung der Kettenlängen, die sich durch ein symmetrisches gaussförmiges Elutionsprofil bei der sterischen Hochleistungsausschlusschromatographie zeigt, und eine homogene Verteilung der geladenen chemischen Gruppen aufweisen, die sich durch ein Elutionsprofil mit einem symmetrischen Maximum bei der Niederdruck-Ionenaustauschchromatographie zeigt,
(2) sowie mindestens einen pharmazeutisch annehmbaren Excipienten,
wobei das Dextranderivat in einer Konzentration unter 10% (w/v) vorliegt.

16. Verwendung der pharmazeutischen Zusammensetzung nach Anspruch 15 zur Herstellung eines Medikaments mit einer Wirkung auf die Narbenbildung der Haut, das zur Verabreichung auf topischem Weg bestimmt ist.

17. Verwendung nach Anspruch 16, **dadurch gekennzeichnet, dass** die pharmazeutische Zusammensetzung in Form einer Paste, einer Salbe, einer wässrigen Flüssigkeit, einer öligen Flüssigkeit, eines wässrigen Gels, eines öligen Gels, eines Aerosols, eines Schaums, einer Mikroemulsion, einer multiplen Emulsion, von Liposomen oder Nanopartikeln vorliegt.

18. Pharmazeutische Zusammensetzung mit antikomplementärer Wirkung, umfassend:
(1) mindestens ein Dextranderivat der allgemeinen Formel DMCₐB_{b}Su_{c}, worin:
D für eine Polysaccharidkette steht, die vorzugsweise aus Aneinanderreihungen von Glucosideinheiten besteht,
MC für Methylcarboxylatgruppen steht,
B für Carboxymethylbenzylamidgruppen steht,
Su für Sulfatgruppen steht (Sulfatisierung der freien Hydroxylfunktionen an den Glucosideinheiten),
a, b und c jeweils für den Grad der Substitution (Sg), ausgedrückt bezogen auf die Anzahl der freien Hydroxylfunktionen in einer Glucosideinheit des Dextrans, in den Gruppen MC, B und Su stehen; wobei a ≥ 0,3; b ≥ 0,1 und c zwischen 0,1 und 0,4 ist,
wobei diese Produkte eine homogene Verteilung der Kettenlängen, die sich durch ein symmetrisches gaussförmiges Elutionsprofil bei der sterischen Hochleistungsausschlusschromatographie zeigt, und eine homogene Verteilung der geladenen chemischen Gruppen aufweisen, die sich durch ein Elutionsprofil mit einem symmetrischen Maximum bei der Niederdruck-Ionenaustauschchromatographie zeigt,
(2) sowie mindestens einen pharmazeutisch annehmbaren Excipienten,
wobei das Dextranderivat in einer Einheitsdosis zwischen 5 und 30 mg vorliegt.

19. Verwendung der pharmazeutischen Zusammensetzung nach Anspruch 18 zur Herstellung eines Medikaments mit antikomplementärer Wirkung.

20. Verwendung nach Anspruch 19, **dadurch gekennzeichnet, dass** die pharmazeutische Zusammensetzung in Form einer isotonischen Lösung vorliegt.

21. Pflaster, **dadurch gekennzeichnet, dass** es mit der pharmazeutischen Zusammensetzung nach Anspruch 15 getränkt ist.
